# EUROPEAN PATENT APPLICATION

(11) **EP 1 867 363 A1**
(43) Date of publication of application: **19.12.2007**
(21) Application number: 06016316.9
(22) Date of filing: 04.08.2006
(51) Int. Cl.: A61Q 11/00, A61K 8/06, A61K 8/33, A61K 8/31, A61K 8/34, A61K 8/92, A61K 8/37

(54) **Antibacterial oral composition.**

(30) Priority: 05.06.2006 IT MI20061091
(71) Applicant: Betafarma S.p.A., 20090 Cesano Boscone (IT)
(72) Inventor: Tallia, Ettore, 20090 Cesano Boscone (Milano) (IT)
(74) Representative: Cicogna, Franco

(57) **Abstract**

Antibacterial toothpaste in the form of an emulsion, comprising an aqueous phase having soluble antibacterial agents dissolved therein, a powder phase, and a third oil phase.

## Description

### BACKGROUND OF THE INVENTION

The present invention relates to an antibacterial composition adapted to be used as a toothpaste for sanitating the oral cavity.

As is known, a not proper and continuous oral hygiene represents, according to recent odontologic and biologic observations, the main cause of different pathologies of the dental apparatus and related mucous-gingival zones.

Paraodontophaties, ulcerative stomatites, tissue necroses, oropharynx cavity halitosis depend, in most cases, on bacterial and fungin infections, persisting for long time periods on the tooth surfaces and adjoining tissues, probably maltreated and hindered in their proper growth.

Today, in most cases, the antibacterial toothpastes designed for providing tooth, gum and mucosa prophylactic hygiene conventionally comprise a homogeneous mixture of two different phases.

A gelled aqueous phase, in which are dissolved one or more antimicrobial substances, and a powder phase including materials with a different and modulated abrading-cleaning power.

In a lot of cases, the antibacterial properties of the above mentioned hygienizing compositions are not sufficiently efficient.

In fact, the contact time between the dental apparatus and toothpaste is usually of a very short duration.

Moreover, the water soluble antiseptic substances containing toothpastes are quickly removed upon rinsing, after their use.

In addition, conventional antibacterial toothpastes, including a water soluble antiseptic substances associated with an oil-soluble antiseptic material dispersed through powders, are quickly removed from a mouth upon a quick rinsing.

### SUMMARY OF THE INVENTION

The aim of the present invention is to provide an emulsified antibacterial toothpaste, designed for sanitating the mouth, while hindering the growth of bacteria and quickly removing bacteria from tooth solid surfaces and mucosa periodontal tissues, thereby overcoming the above mentioned drawbacks.

Within the scope of the above mentioned aim, a main object of the invention is to provide such a toothpaste having a long duration sanitation activity.

According to the present invention, novel antibacterial toothpastes are herein provided, which are stably emulsified, and comprise therein specifically designed "lipophyl complex".

In the above mentioned high substantiveness oil complex formulations, for tooth and gum tissues, antiseptic substances, which are only soluble in an oil phase, can be dissolved.

Thus, a novel toothpaste composition has been provided, with the addition of a third oil phase, which implements the two phases (i.e. the aqueous and powder phases) of conventional toothpastes.

Thus, it is possible to form through the overall oral cavity and in particular on the teeth, an oil film, which is resistant against water rinsing, and adapted to provide a long duration sanitation activity of the antibacterial substances contained in the compositions.

Moreover, the provision, in each individual toothpaste composition, of two antimicrobial substances dissolved in different phases (aqueous-oil), is such as to synergically enhance their individual biologic properties.

### EXAMPLE

A toothpaste in an emulsified paste-cream form, in which the three component phases, i.e. the aqueous phase, powder phase and oil phase, have been added with oil and water-soluble antiseptic substances (dissolved in oil and water) has been prepared.

The emulsion composition can be based on different ratios of the three aqueous, powder and oil phases.

The aqueous phase can vary from about 40% w/w to about 10% w/w.

In addition to the main component, i.e. water, this phase can be supplemented by other water soluble substances such as moistening agent (glycerol, sorbitol, xylitol, glycols) fluorinated salts, sweetening substances (saccharine, aspartames), coloring substances, pH adjusters, cleansing foamed substances, antiplaque substances, (zinc salts such as citrates).

In this phase, an emulsifying system of the type oil in water (O/W) and a water soluble antibacterial substances are always present.

The powder phase comprises materials having a different and modulated abrading-cleaning powder, like those which are usually used as toothpaste fillers (silicon dioxide, aluminium dioxide, calcium phosphates, calcium carbonate).

The powder phase can vary from about 5% w/w to about 30% w/w.

The oil phase can vary from about 3% w/w to about 25% w/w.

The oil phase can comprise vegetal oils, mineral oils, aliphatic esters, aliphatic ethers, aliphatic alcohols, triglygerides and aliphatic hydrocarbons.

The composition according to the invention can also comprise aromatizing substances and oil-soluble antibacterial substances.

It has been found that the composition according to the present invention is adapted to sanitate the mouth, by hindering the growth of bacteria and satisfactorily removing bacteria from the dental solid surfaces and periodontal mucosa tissues.

## Claims

1. An antibacterial composition, particularly to be used as a toothpaste, for sanitating the oral cavity, said antibacterial composition comprising antiseptic substances and being **characterized in that** it further comprises at least an oil phase.

2. A composition according to claim 1, **characterized in that** said composition comprises an emulsion and a further aqueous phase, in which said antibacterial substances, comprising water soluble antibacterial substances, are dissolved.

3. A composition according to claim 2, **characterized in that** said aqueous phase varies from about 10% w/w to about 40% w/w.

4. A composition according to claim 1, **characterized in that** said composition comprises water soluble substances selected from moistening agents, alcohols and glycols, fluorinated salts, sweetening agents, coloring agents, pH adjusters, cleaning foamed agents.

5. A composition according to claim 4, **characterized in that** said moistening agents are selected from glycerol, sorbitol, xylitol, glycols and said sweetening agents are selected from saccharin and aspartames.

6. A composition according to claim 1, **characterized in that** said oil phase varies from about 3% w/w to about 25% w/w.

7. A composition according to claim 1, **characterized in that** said oil phase comprises a substances selected from vegetal oils, mineral oils, aliphatic esters, aliphatic ethers, aliphatic alcohols, triglycerides and aliphatic hydrocarbons

8. A composition according to claim 1, **characterized in that** said composition comprises oils and aromatizing substances.
